# EUROPEAN PATENT SPECIFICATION

(11) **EP 0 589 566 B1**
(45) Date of publication and mention of the grant of the patent: **21.06.2000**
(21) Application number: 93306555.9
(22) Date of filing: 19.08.1993
(51) Int. Cl.: A61M 25/00, A61M 29/02

(54) **Surgical device permitting the modification in vivo of its mechanical properties**
Chirurgisches Gerät, das die Änderung seiner mechanischen Eigenschaften invivo ermöglicht
Dispositif chirurgical permettant de modifier in vivo ses propriétés mécaniques

(30) Priority: 20.08.1992 US 932607
(43) Date of publication of application: 30.03.1994
(73) Proprietor: ADVANCED CARDIOVASCULAR SYSTEMS, INC., Santa Clara California 95052-8167 (US)
(72) Inventor: Muni, Ketan P., San Jose, California 95136 (US)
(74) Representative: McLeish, Nicholas Alistair Maxwell

(56) References cited:
- US-A- 4 624 657
- PATENT ABSTRACTS OF JAPAN vol. 16, no. 533 (C-1002) 04 November 1992 & JP-A-04 200 565 (OLYMPUS OPTICAL CO. LTD.) 21 July 1992
- PATENT ABSTRACTS OF JAPAN vol. 16, no. 349 (C-0967) 28 July 1992 & JP-A-04 105 660 (OLYMPUS OPTICAL CO. LTD) 07 April 1992

## Description

### BACKGROUND OF THE INVENTION

### Field of the Invention

The present invention relates generally to angioplastic procedures and more particularly pertains to surgical devices that are introduced into and advanced through the vascular system during angioplastic procedures.

### Description of the Prior Art

A variety of devices are employed in modern angioplastic techniques that are intended for introduction into and advancement through a patient's vasculature. Such devices include guidewires, various catheter type structures, retrieval devices, stents, etc. An important criterion inherent in the design and construction of such devices is the ability of the device to be easily advanced through vessels to the operative site and furthermore, to provide such "transportability" without compromising the ability of the device to perform its intended function once in position. The relatively small diameter of the vessels through which such devices are routinely passed and the potentially long and tortuous paths such devices may be forced to follow renders certain mechanical properties of such devices of critical importance.

The rigidity of an intravascular device is a representative example of a mechanical property of crucial import vis-a-vis the transportability of the device. The pushability, torqueability, and trackability of an elongated structure are all affected by the inherent rigidity of that structure. A guidewire or catheter must, for example, have sufficient rigidity to enable its distal end to be maneuvered by manipulation of its proximal end. Conversely, such a device must be sufficiently flexible to enable it to follow a circuitous path and to prevent injury to the vessel walls.

The cross-sectional dimensions or profile of an intravascular device are dictated by the size of the vessels through which the device is to be advanced. In the case of balloon catheters, the mechanical properties of the materials employed in the device's construction play an important role in the ability of the device to be initially constrained to a sufficiently small profile in order to facilitate its advancement from the point of introduction to the operative site, to readily assume a substantially larger size upon expansion at the operative site, and to subsequently reassume the initial small profile for unrestricted extraction from the patient. It is therefore essential to select a material for the construction of a balloon that is flexible, strong and has a "memory."

Some of these seemingly mutually exclusive requirements have been previously addressed by innovative approaches. With respect to the above-described requirements for guidewires or catheters to be rigid for some purposes yet simultaneously flexible for other reasons, it was recognized that a flexible distal end matched with a relatively rigid body element would substantially enhance the transportability of such a device. However, the utility of such an approach is nonetheless limited as situations inevitably will be encountered where the distal end of a particular device is either too stiff to be forced through a small radius bend, or too flexible to allow its position to be properly controlled. Furthermore, a very flexible tip may be necessary in order to reach a targeted stenosis, while substantially more stiffness may be required in order to cross through the stenosis if particularly occluded.

With regard to the above-described problems vis-a-vis the profile of balloon catheters, it has been found that by folding the balloon material into certain configurations a smaller deflated profile can be achieved for a given inflated size. However, the balloon's ultimate strength and the ability of a balloon to "remember" its folded shape typically are properties that are in conflict with one another, i.e. the stronger, more rupture-resistant the material, the weaker its memory.

The transportability of a prior art intravascular device may be hindered under certain conditions by the very mechanical properties of the materials employed in its construction that serve to enhance the device's transportability under a different set of conditions. Similarly, those mechanical properties of the materials that optimize the device's intended performance once in position may severely compromise its transportability. The prior art has provided for only limited improvements in this regard by typically employing either a specializing approach, i.e., by optimizing relevant parameters to fit a specific situation, or a compromising approach, i.e., providing somewhat compromised performance but for a wider range of situations. The capability for adapting a specific angioplastic device to different situations at different times has not been provided by the prior art.

What has been provided however, is a stent formed from a shape memory resin which softens when heated. The stent, which is described in JP-A-4,200,565, is mounted on a balloon catheter and is provided on an inner surface with a layer of heat accumulating material. Upon inflation of the balloon catheter the heat accumulating material is pressurised causing it to change from the liquid phase to the solid phase and generate heat. This heat softens the stent allowing it to increase in diameter. Once the phase change is complete the heat accumulating material stops generating heat and the stent cools and is cured to maintain its larger diameter configuration.

### SUMMARY OF THE INVENTION

According to the present invention there is provided an angioplastic surgical device for insertion into and transport through a patient's vascular system, comprising an elongate member having a selected region formed of material having mechanical properties subject to change upon variation of said material's operating temperature, characterised in that the surgical device further comprises control means for varying at a controllable rate the operating temperature of the selected region between a plurality of selected temperatures other than ambient and which are between limits tolerable within the body, thus controlling the mechanical properties of the material.

Embodiments of the present invention serve to substantially enhance the transportability of intravascular devices by providing for the controllable variation of some of the mechanical properties of such devices while situated within a patient's vasculature. This is achieved by employing carefully selected materials in the construction of critical components or regions of an intravascular device and by providing means therein for remotely varying its operating temperature. The materials are selected to have certain mechanical properties that change as their temperature is varied between limits tolerable within the body. Embodiments of the present invention encompass both the cooling as well as the heating of the device by a variety of means while it is situated within the patient. Moreover, the invention is adaptable to many different intravascular devices.

A guidewire or catheter constructed according to one embodiment of the present invention has a distal end formed of material selected to become increasingly flexible with increased temperature. The material is further selected to display a desirable range of stiffness within the range of temperatures tolerable within the body. The present invention provides for a variety of ways to controllably increase the temperature of the distal end while situated within the vascular system. In a preferred embodiment, electrical current passed through a resistive element incorporated in or comprising the distal end of the device serves to quickly and very controllably raise the temperature. Alternatively, energy absorptive materials are incorporated in the distal end of the device to absorb energy radiated by an external electromagnetic energy source or generated by an oscillating magnetic field. As a further alternative, light energy is conducted to the distal end via an optical fiber where it is absorbed by material forming the distal end. In yet a further embodiment lumen structures formed within certain angioplastic devices are alternatively employed to conduct heated fluid to and from selected regions of the device wherein an increased temperature is desired.

Embodiments of the present invention additionally, optionally provide for the cooling of selected portions of surgical devices either to lower their operating temperature to below ambient or to more rapidly reduce their previously elevated temperature. This is accomplished either by conducting cooled fluid or gas to and from the selected region of the surgical device, allowing a liquid conducted thereto to undergo a phase change or allowing a compressed gas conducted thereto to expand. Alternatively, a Peltier- type device is incorporated in the selected region in order to facilitate a lowering of the temperature via electronic means.

The temperature of the region of the device subject to heating and/or cooling is optionally monitored. A thermocouple, an optical sensor or a resistance device, for example, is readily adaptable to provide a real time, accurate indication of the temperature of a specific component or region of the surgical device while situated within the vascular system. The sensed temperature data is further optionally utilized in a closed loop control system to facilitate an automatic control of a desired set temperature.

During the angioplastic procedure, the progress of the distal end of the device through the vascular system is monitored in the conventional manner. In the case of a guidewire or catheter, when additional flexibility is required in order to negotiate a particularly circuitous path, the temperature of the device is increased. Conversely, when more pushability is required of the device, its temperature is allowed to decrease back down to ambient or is actively reduced by cooling means. In the case of a foldable balloon catheter, upon completion of the dilatation of the targeted artery, the balloon is collapsed and its temperature is increased to promote its folding after which the device is more easily extracted from within the vascular system.

These and other features and advantages of the present invention will become apparent from the following detailed description of some preferred embodiments which, taken in conjunction with the accompanying drawings, illustrates by way of example the principles of the invention.

### BRIEF DESCRIPTION OF THE DRAWINGS

FIGURE 1 is an enlarged longitudinal cross-sectional view of a guidewire device embodying the present invention.
FIG. 2 is an enlarged longitudinal cross-sectional view of a catheter device embodying to the present invention.
FIG. 3 is an enlarged longitudinal cross-sectional view of a balloon dilatation catheter embodying to the present invention.
FIG. 4A is a cross-sectional view of FIG. 3 taken along lines 4A-4A.
FIG. 4B is a cross-sectional view of the device of FIG. 4A shown in its folded state.
FIG. 5 is an enlarged longitudinal cross-sectional view of an alternative embodiment catheter device.
FIG. 6 is an enlarged longitudinal cross-sectional view of a further alternative embodiment catheter device.

### DETAILED DESCRIPTION OF PREFERRED EMBODIMENTS

The Figures illustrate various embodiments of the present invention. Each embodiment comprises an angioplastic surgical device which is inserted into a patient's vascular system, maneuvered to the operative site, and ultimately removed at the conclusion of the procedure. The present invention provides for an enhancement of the transportability of such devices throughout the vascular system.

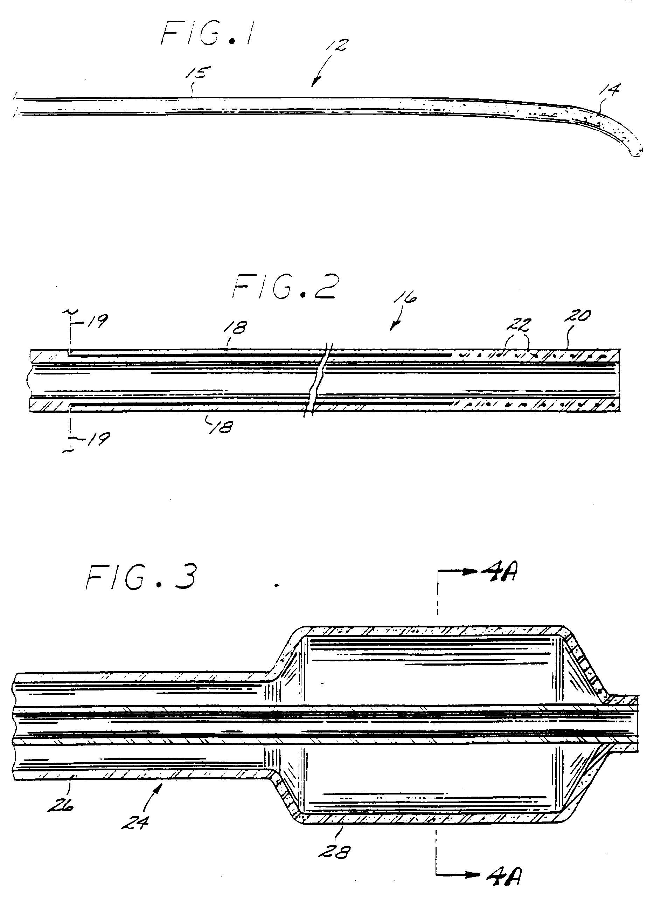

FIG. 1 illustrates an intravascular guidewire 12 embodying the present invention. Such devices are employed to establish a route from the point of entry to the operative site. A curved distal end allows the wire to be maneuvered through portions of the vascular system by pushing and applying torque to the proximal end at the appropriate moments. Once in place the wire serves as a track for other devices that are advanceable along its length.

In accordance with a prefered embodiment of the present invention, the distal end 14 of the guidewire is constructed of material that readily absorbs radiated energy and becomes increasingly more flexible with an increase in its temperature. In the preferred embodiment, the shaft portion 15 of the guidewire is formed of a non-ferrous metal while the distal end 14 is formed of ferrous metal and brazed, soldered or welded to or otherwise attached to the shaft portion.

In use, the guidewire is introduced into the vascular system and manipulated in the conventional manner in an effort to reach the operative site until an otherwise impassible tortuosity is encountered whereby the inherent rigidity of the distal end 14 prevents the guidewire from being extended further. At such point, the distal end is subjected to a strong magnetic field oscillating at a high frequency by energizing a magnetic field generator (not shown) in proximity to the patient. The oscillating field causes the ferrous distal end 14 to heat up and become increasingly flexible. Once sufficient flexibility is achieved, the guidewire can be extended through previously impassible tortuosity. The distal end 14 is preferably formed of a coil or many parallel wires embedded in a matrix such as wax, the mechanical properties of which coil or wires change drastically with temperatures. The coil or set of wires is welded to a shaft formed of stainless steel. A magnetic field of about 100 Gauss, oscillating at 100 KHz to 10 MHz provides for the rapid heating of the distal end.

FIG. 2 illustrates a catheter 16 embodying the present invention. Such catheter is advanceable along a previously emplaced guidewire to the operative site within a patient's vascular system. The catheter is formed of material that becomes substantially more flexible as its temperature is increased above the patient's ambient temperature (nominally 98.6°F or 37°C) to a temperature of 60°C which has been found to be tolerable within the body for limited periods of time. In the embodiment illustrated in FIG. 2, an electrical conductor 18 is incorporated in the catheter wall. The two leads 19 of the conductor are inter-connectable to a power supply (not shown) near the catheter's proximal end and are linked to resistance wiring 22 coiled about the distal end 20. Passing electric current through the wiring causes the temperature of the distal end to rise at a very controllable and predictable rate resulting in an increase in the flexibility of the distal end 20.

In use, the catheter 12 is advanced along an emplaced guidewire until its progress is impeded by an excessively tight bend in the guidewire. In order to facilitate further progress and prevent injury to the vessel walls, an electric current is passed through conductor 18 and resistance wiring 22 in order to increase the operating temperature of the distal end 20. Once past the hinderance, the temperature of the distal end is reduced by decreasing power or supply of electrical current.

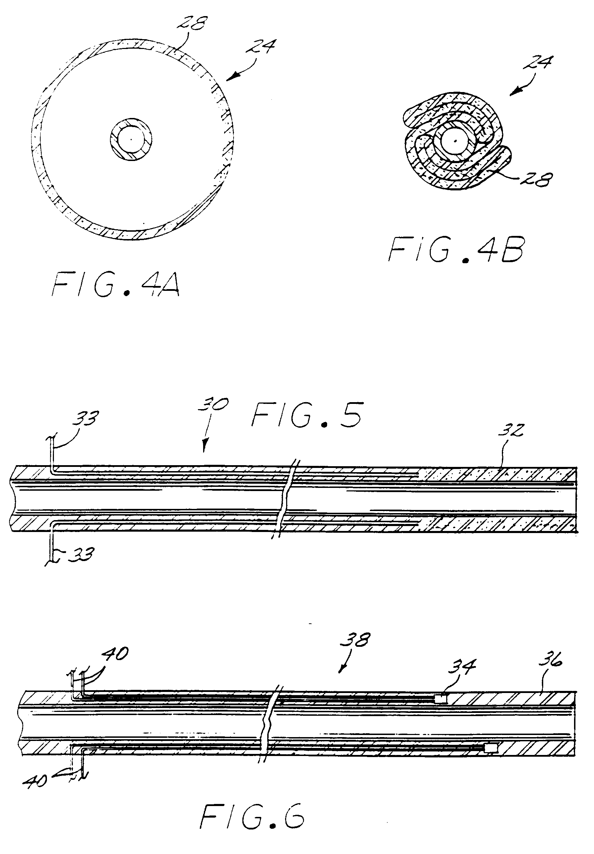

FIG. 3 illustrates a balloon-type dilatation catheter 24. The device consists of a multiple lumen catheter body 26 having an inflatable balloon structure 28 near its distal end. While the balloon is in its collapsed state, the device is advanceable along a previously emplaced guidewire to the operative site. Dilatation of the targeted artery is accomplished upon inflation of the balloon via fluid pumped through one of the lumens. Deflation of the balloon at the conclusion of the operation allows the device to be withdrawn. In accordance with a prefered embodiment of the present invention, the material forming the balloon is capable of absorbing energy from an externally positioned radiation source. Alternatively, the material forming the balloon has embedded therein energy-absorbing particles. Absorption of energy causes the balloon material's temperature to rise which renders the balloon more readily apt to reassume its folded configuration. FIG. 4A illustrates the balloon in its inflated state while FIG. 4B illustrates a folded configuration that has been found to provide an especially compact profile.

In use, the folded balloon catheter is first advanced along a guidewire extending to the stenosed artery. Inflation of the balloon causes the artery to be dilated after which an externally positioned radiation source is energized to heat the balloon while the balloon is being deflated. The balloon reassumes its tightly folded configuration and is then readily withdrawn from within the patient's vascular system.

The balloon is preferably formed of polyethylene terephthalate ("PET") or nylon having ferrite particles embedded therein. Such particles readily absorb energy radiated by a radio frequency electromagnetic radiation source operating at about 100 KHz to 100 MHz. Alternatively, the balloon is formed of plastic with large dielectric loss tangent which absorbs microwave electromagnetic energy at about 36 Hz to 10 MHz. Electromagnetic energy sources with an output of about 100 Watts have been found to be quite effective at a distance of 10 centimeters outside the body and generally adjacent the operative site.

The balloon catheter 24 shown in FIG. 3 alternatively is heated with the introduction of heated fluid just prior to the final deflation and withdrawal. The balloon 28 may be made of a material which is affected by changes in its operating temperature, for example cross linked polymers or thermoplastics such as polyethylene terephthalate. The heated fluid serves to transfer heat to the balloon 28 which in turn renders the balloon more pliable and therefore more readily disposed to reassume its folded shape (FIG. 4B). In the event enhanced rupture strength is required during dilatation, the balloon is expanded with cooled fluid.

FIG. 5 illustrates another alternative embodiment of a catheter 30. The distal end 32 of the surgical device is formed of material that becomes more flexible with an increase in temperature. Moreover, the material is selected to absorb light energy at a preselected wavelength. One or more optical fibers 33 are incorporated in the catheter wall to conduct light of the preselected wavelength from a laser (not shown) to the distal end 32 of the catheter 30. Energization of the laser causes the distal end 32 to heat up.

FIG. 6 illustrates an alternative embodiment wherein a plurality of Peltier-type devices 34 are incorporated near the distal end 36 of a catheter 38. By energizing leads 40 with the appropriate voltage and polarity, the distal end 36 of the catheter is cooled. In use, when an increase in stiffness is necessary, as for example when it is necessary to push the catheter 38 through an occlusion in an artery, the Peltier-type devices 34 are energized to quickly and predictably lower the temperature and increase stiffness. Temperatures near 0°C are tolerable within the vascular system for brief periods of time.

While particular embodiments of the invention have been illustrated and described, it will also be apparent to those skilled in the art that various modifications can be made without departing from the scope of the invention. For example, a wide variety of materials will be adaptable for use in devices embodying to the present invention. Additionally, many different well-known methods can be employed to bring about an increase or a decrease of the surgical device's operating temperature. Furthermore, it is not intended that the present invention is to be limited to the heating or cooling of the distal end of the various angioplastic surgical devices, as other regions of such devices may benefit from a change in their mechanical properties as brought about by the heating or cooling thereof.

## Claims

1. An angioplastic surgical device (12,16,24,30,38) for insertion into and transport through a patient's vascular system, comprising an elongate member (15,26)having a selected region (14,20,28,32,36) formed of material having mechanical properties subject to change upon variation of said material's operating temperature, characterised in that the surgical device (12,16,24,30,38) further comprises control means (22,33,34) for varying at a controllable rate the operating temperature of the selected region (14,20,28,32,36) between a plurality of selected temperatures other than ambient and which are between limits tolerable within the body, thus controlling the mechanical properties of the material.

2. The device of claim 1, wherein the device comprises an angioplastic catheter (16), the selected region comprises a distal end (20) of the catheter (16) and the material of which the distal end (20) is formed becomes more flexible as its operating temperature is increased.

3. The device of claim 1, wherein the device comprises a balloon catheter (24), the selected region comprises an inflatable meter (28) and the material of which the inflatable member (28) is formed assumes an increasingly tightly folded configuration as its temperature is increased and gains rupture strength as its temperature is decreased.

4. The device of claim 1, wherein the device comprises a surgical guidewire (12), the selected region comprises a distal end (14) of the guidewire (12) and the material of which the distal end (14) is formed becomes more flexible as its operating temperature is increased.

5. The device of claim 4, wherein said material is a ferrous metal and said control means comprises means for exposing the distal end (14) to a magnetic field.

6. The device of any of claims 1 to 5, wherein said control means includes heating means for increasing the operating temperature of the selected region.

7. The device of claim 6, wherein said heating means includes a resistive heating element (22) for generating heat when energised by an electric current.

8. The device of claim 6, wherein said heating means includes means for absorbing radiated electromagnetic energy such that the operating temperature of the selected region is increased when such electromagnetic energy is absorbed.

9. The device of claim 8, wherein said heating means includes means for absorbing electromagnetic radiation of microwave frequencies.

10. The device of claim 8, wherein said heating means includes means for absorbing electromagnetic radiation of radio frequencies.

11. The device of claim 6, wherein said heating means includes means for absorbing energy generated by an oscillating magnetic field.

12. The device of claim 6, wherein said heating means includes an optical fiber (33) and means for absorbing energy transmitted through the optical fiber.

13. The device of claim 6, wherein said heating means includes a heated fluid in contact with the selected region.

14. The device of any of any preceding claim, wherein said control means includes cooling means (34) for decreasing the operating temperature of the selected region.

15. The device of claim 14, wherein said cooling means includes a Peltier-type device (34).

16. The device of claim 14, wherein said cooling means includes a cooled fluid in contact with the selected region.

## Patentansprüche

1. Angioplastische chirurgische Vorrichtung (12,16,24,30,38) zum Einsetzen in und Transport durch das Gefäßsystem eines Patienten, umfassend ein langgestrecktes Element (15,26), das einen gewählten Bereich (14,20,28,32,36) aufweist, der aus einem Material mit mechanischen Eigenschaften gebildet ist, die sich bei Änderung der Betriebstemperatur des Materials ändern, dadurch gekennzeichnet, dass die chirurgische Vorrichtung (12,16,24,30,38) ferner Steuermittel (22,33,34) umfaßt, um die Betriebstemperatur des gewählten Bereichs (14,20,28,32,36) zwischen einer Mehrzahl gewählter Temperaturen, die außerhalb der Umgebungstemperatur und zwischen im Körper tolerierbaren Grenzen liegen, mit einer steuerbaren Rate zu ändern, um hierdurch die mechanischen Eigenschaften des Materials zu steuern.

2. Vorrichtung nach Anspruch 1, wobei die Vorrichtung einen angioplastischen Katheter (16) aufweist, wobei der gewählte Bereich ein distales Ende (20) des Katheters (16) aufweist und wobei das Material, aus dem das distale Ende (20) gebildet ist, flexibler wird, wenn seine Betriebstemperatur zunimmt.

3. Vorrichtung nach Anspruch 1, wobei die Vorrichtung einen Ballonkatheter (24) aufweist, wobei der gewählte Bereich ein aufpumpbares Element (28) aufweist, und wobei das Material, aus dem das aufpumpbare Element (28) gebildet ist, eine zunehmend eng gefaltete Konfiguration einnimmt, wenn seine Temperatur erhöht wird, und an Rissfestigkeit gewinnt, wenn seine Temperatur gesenkt wird.

4. Vorrichtung nach Anspruch 1, wobei die Vorrichtung einen chirurgischen Führungsdraht (12) aufweist, wobei der gewählte Bereich ein distales Ende (14) des Führungsdrahts (12) aufweist und wobei das Material, aus dem das distale Ende (14) gebildet ist, flexibler wird, wenn seine Betriebstemperatur zunimmt.

5. Vorrichtung nach Anspruch 4, wobei das Material ein Eisenmaterial ist und das Steuermittel Mittel aufweist, um das distale Ende (14) einem Magnetfeld auszusetzen.

6. Vorrichtung nach einem der Ansprüche 1 bis 5, wobei das Steuermittel Heizmittel aufweist, um die Betriebstemperatur des gewählten Bereichs zu erhöhen.

7. Vorrichtung nach Anspruch 6, wobei das Heizmittel ein Widerstandsheizelement (22) umfaßt, um bei Erregung durch elektrischen Strom Wärme zu erzeugen.

8. Vorrichtung nach Anspruch 6, wobei das Heizmittel Mittel umfaßt, um abgestrahlte elektromagnetische Energie zu absorbieren, derart, dass die Betriebstemperatur des gewählten Bereichs zunimmt, wenn derartige elektromagnetische Energie absorbiert wird.

9. Vorrichtung nach Anspruch 8, wobei das Heizmittel Mittel umfaßt, um elektromagnetische Strahlung mit Mikrowellenfrequenzen zu absorbieren.

10. Vorrichtung nach Anspruch 8, wobei das Heizmittel Mittel umfaßt, um hochfrequente elektromagnetische Strahlung zu absorbieren.

11. Vorrichtung nach Anspruch 6, wobei das Heizmittel Mittel umfaßt, um durch ein oszillierendes Magnetfeld erzeugte Energie zu absorbieren.

12. Vorrichtung nach Anspruch 6, wobei das Heizmittel eine optische Faser (33) und Mittel umfaßt, um durch die optische Faser übertragene Energie zu absorbieren.

13. Vorrichtung nach Anspruch 6, wobei das Heizmittel ein erwärmtes Fluid in Kontakt mit dem gewählten Bereich umfaßt.

14. Vorrichtung nach einem der vorhergehenden Ansprüche, wobei das Steuermittel ein Kühlmittel (34) umfaßt, um die Betriebstemperatur des gewählten Bereichs zu senken.

15. Vorrichtung nach Anspruch 14, wobei das Kühlmittel eine Peltier-Vorrichtung (34) umfaßt.

16. Vorrichtung nach Anspruch 14, wobei das Kühlmittel ein gekühltes Fluid in Kontakt mit dem gewählten Bereich umfaßt.

## Revendications

1. Dispositif chirurgical angioplastique (12, 16, 24, 30, 38) pour insertion dans un système vasculaire d'un patient et transport à travers celui-ci, comprenant un élément de forme allongée (15, 26) ayant une région sélectionnée (14, 20, 28, 32, 36) formée d'un matériau ayant des propriétés mécaniques soumises à des changements lors de la variation de la température de fonctionnement dudit matériau, caractérisé en ce que le dispositif chirurgical (12, 16, 24, 30, 38) comprend en outre un moyen de commande (22, 33, 34) pour faire varier à une vitesse commandable la température de fonctionnement de la région sélectionnée (14, 20, 28, 32, 36) entre une pluralité de températures sélectionnées autres que la température ambiante et qui sont situées entre des limites tolérables à l'intérieur du corps, commandant ainsi les propriétés mécaniques du matériau.

2. Dispositif selon la revendication 1, dans lequel le dispositif comprend un cathéter angioplastique (16), la région sélectionnée comprend une extrémité distale (20) du cathéter (16) et le matériau avec lequel l'extrémité distale (20) est formée devient plus flexible à mesure que sa température de fonctionnement est accrue.

3. Dispositif selon la revendication 1, dans lequel le dispositif comprend un cathéter (24) à ballonnet, la région sélectionnée comprend un élément gonflable (28) et le matériau avec lequel l'élément gonflable (28) est formé prend une configuration fortement repliée de manière croissante à mesure que sa température est accrue et augmente sa résistance à la rupture à mesure que sa température est diminuée.

4. Dispositif selon la revendication 1, dans lequel le dispositif comprend un câble de guidage chirurgical (12), la région sélectionnée comprend une extrémité distale (14) du câble de guidage (12) et le matériau avec lequel l'extrémité distale (14) est formé devient plus flexible à mesure que sa température de fonctionnement est accrue.

5. Dispositif selon la revendication 4, dans lequel ledit matériau est un métal ferreux et ledit moyen de commande comprend un moyen pour exposer l'extrémité distale (14) à un champ magnétique.

6. Dispositif selon l'une des revendications 1 à 5, dans lequel ledit moyen de commande inclut un moyen de chauffage pour accroître la température de fonctionnement de la région sélectionnée.

7. Dispositif selon la revendication 6, dans lequel ledit moyen de chauffage inclut un élément de chauffage résistif (22) pour produire de la chaleur lorsque alimenté par un courant électrique.

8. Dispositif selon la revendication 6, dans lequel ledit moyen de chauffage inclut un moyen pour absorber l'énergie électromagnétique rayonnée d'une manière telle que la température de fonctionnement de la région sélectionnée est accrue lorsqu'une telle énergie électromagnétique est absorbée.

9. Dispositif selon la revendication 8, dans lequel ledit moyen de chauffage inclut un moyen pour absorber le rayonnement électromagnétique des hyperfréquences.

10. Dispositif selon la revendication 8, dans lequel ledit moyen de chauffage inclut un moyen pour absorber le rayonnement électromagnétique des fréquences radio.

11. Dispositif selon la revendication 6, dans lequel ledit moyen de chauffage inclut un moyen pour absorber l'énergie produite par un champ magnétique oscillant.

12. Dispositif selon la revendication 6, dans lequel ledit moyen de chauffage inclut une fibre optique (33) et un moyen pour absorber l'énergie transmise à travers la fibre optique.

13. Dispositif selon la revendication 6, dans lequel ledit moyen de chauffage inclut un fluide chauffé en contact avec la région sélectionnée.

14. Dispositif selon l'une des revendications précédentes, dans lequel ledit moyen de commande inclut un moyen de refroidissement (34) pour diminuer la température de fonctionnement de la région sélectionnée.

15. Dispositif selon la revendication 14, dans lequel ledit moyen de refroidissement inclut un dispositif du type à effet Peltier (34).

16. Dispositif selon la revendication 14, dans lequel ledit moyen de refroidissement inclut un fluide refroidi en contact avec la région sélectionnée.
